(19) Europäisches Patentamt
European Patent Office
Office européen des brevets

(11) EP 4 390 943 A1

(12) EUROPEAN PATENT APPLICATION

(43) Date of publication:
26.06.2024 Bulletin 2024/26

(51) International Patent Classification (IPC):
G16C 60/00 (2019.01)     G06N 3/00 (2023.01)

(21) Application number: 22215223.3

(22) Date of filing: 20.12.2022

(52) Cooperative Patent Classification (CPC):
G16C 60/00; G06N 3/0464; G06N 3/08; G06N 5/01;
G06N 20/20; G06N 3/048; G16C 20/30;
G16C 20/70

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: Borealis AG
1020 Vienna (AT)

(72) Inventors:
• TORRES, Juan Pablo
4021 Linz (AT)

• JERABEK, Michael
4021 Linz (AT)
• KIEHAS, Florian
4040 Linz (AT)
• REITER, Martin
4040 Linz (AT)

(74) Representative: Ter Meer Steinmeister & Partner
Patentanwälte mbB
Nymphenburger Straße 4
80335 München (DE)

(54) **PREDICTION OF THE DUCTILE-TO-BRITTLE TRANSITION TEMPERATURE OF POLYMER COMPOSITIONS BASED ON IMPACT CURVES**

(57)     The present invention relates to a computer implemented method for predicting the ductile-to-brittle transition temperature of a test polymer composition based on impact curves. Furthermore, a non-transitory computer readable storage medium is provided for tangibly storing computer program instructions capable of being executed by a processor, the computer program instructions defining the steps the aforementioned computer implemented method. Furthermore, the invention is directed to the use of impact curves and values indicative of the ductile-to-brittle transition temperatures of polymer compositions for training machine learning algorithms.

Fig. 1

## Description

### Field of the invention

The present invention relates to a computer implemented method for predicting the ductile-to-brittle transition temperature of a test polymer composition based on impact curves. Furthermore, a non-transitory computer readable storage medium is provided for tangibly storing computer program instructions capable of being executed by a processor, the computer program instructions defining the steps the aforementioned computer implemented method. Furthermore, the invention is directed to the use of impact curves and ductile-to-brittle transition temperatures of polymer compositions for training a machine learning algorithm.

### Technical background

Polymers exhibit a transition in their capacity to absorb energy when subjected to impact loads. Under predefined test conditions, there will be a temperature below which a polymer will manifest brittle behavior. In other words, it will break under influence of a rather small amount of energy. Above this temperature, the same polymer will respond in a ductile manner and will be able to absorb a comparatively high amount of energy before failing. On a microscopic level, brittle failure is characterized by a sharp, clean break with little or no molecular displacement. In contrast, ductile failure is accompanied by yielding, *i.e.* onset of plastic deformation in vicinity of the impact position. These phenomena are observed for both polymers and polymer compositions without exceptions.

The temperature at which the above outlined transition from the ductile to the brittle behavior takes place is referred to as the ductile-brittle transition temperature (DBTT). For many applications, the DBTT is a key material parameter. Polymer compositions for automotive applications are, for example, often required to have low DBTTs. Additionally, tailoring polymer compositions with respect to their DBTT is especially crucial for engineering applications in which the expected temperature covers a wide range. Up to now, development of polymer compositions for such applications involves extensive experimentation. In order to determine the DBTT, the toughness of the polymer compositions needs to be measured over a wide range of temperatures resulting in a high number of experiments that have to be conducted.

This experimental effort could be reduced if a computer-implemented method would be provided that is able to predict the DBTT of a composition on the basis of a few, already available experimental data. Such a method for predicting the ductile-to-brittle transition temperature would likely also be beneficial because it would reduce expenditures and would allow to accelerate the development of new polymer compositions.

Generally, computer-implemented methods for predicting material properties are known in the prior art.

WO 2021 / 110 690 A1, for example, discloses a computer-implemented method for determining a material property, for example Young's modulus, from a representation of a foam sample, for example an image of a foam sample. To that end, a structural feature, especially a microscopic feature related to a pore, wall, cell, strut or node, is derived from the image of the foam sample in a first step. This structural feature is then provided to a material model such as a physical model or a data-driven model. The data-driven model may be parametrized based on training data and may be capable of correlating the input structural features to output material properties without involving any knowledge of laws of physics. Whereas this might be a viable model for foam samples, it cannot be tranferred to other kinds of materials.

Furthermore, US 10,955,362 B2 describes a computer-implemented method for determining the quality of polymers. To that end, a Raman spectrum of a polymer sample is obtained and polymer properties/features are computed by comparing the chemical and/or structural fingerprint to previously stored properties data using a machine learning tool. The algorithms of the machine learning tool are trained with historical Raman data and polymer properties/features of known samples measured in a laboratory with conventional methods. For a desired polymer property, a data set - including the measured polymer properties/features and the respective Raman spectral data is ingested into a database accessible via an application programming interface, API. The method provides reasonable predictions for polymer properties such as the MFR and the ethylene content. Yet, it is not comparably successful in predicting arbitrary polymer properties.

Additionally, a laser has to be used in order to obtain Raman spectra. Hence, generation of the input data is associated with high costs for the mesurement equipment and also poses high demands to ensure safety of the operators.

Thus, in view of the above, no computer-implemented method is available that would be able to provide accurate predictions of the DBTT for polymer compositions. Hence, the abovementioned need to provide such a method persists.

### Summary of the Invention

The above identified need is satisfied by the computer implemented method according to independent claim 1 and the use of impact curves and values indicative of ductile-to-brittle transition temperatures in machine learning according to claim 15. Advantageous embodiments may be derived from the dependent claims.

The computer-implemented method for predicting the ductile-to-brittle transition temperature (DBTT) of a test polymer composition comprises at least three steps. In a first step, a machine learning algorithm is trained with training polymer compositions (hereinafter also "training phase"). This is done by mapping data sets extracted from impact curves of the training polymer compositions to values indicative of DBTTs of the training polymer compositions. In a second step, the trained algorithm is fed, as an input, with corresponding data set(s) extracted from at least one impact curve of the test polymer composition. A third step of the method comprises receiving, as an output, a value indicative of the DBTT of the test polymer composition. The second and third step may, therefore, also be referred to as the "prediction phase".

This method proved to be a powerful asset in material research and development. Most notably, the value indicative of the DBTT, which is received or provided as an output, is an important material property. It is especially important for polymer compositions, above all for polymer compositions that are used for the manufacture of load-bearing elements. Traditionally, however, the determination of values indicative of the DBTT has been quite time consuming and involved extensive use of specialized measurement equipment. More specifically, impact tests or fracture tests of test material had to be carried out at a variety of different temperatures in order to derive values indicative of the DBTT. The inventive method, in contrast to that, is able to deliver these values within seconds or a few minutes based on a data set derived from a single experiment at a constant temperature. Hence, the inventive method is a time-efficient alternative to the conventional experimental approach and it does not rely on excessive use of valuable measurement equipment, material and/or energy resources.

These advantages may be considered the reward for the effort which is invested in the first step, *i.e.* the training of the machine learning algorithm. To ensure that the initially invested effort is outweighed by the advantages, it is preferred when the method according to the present invention is used in an environment in which new polymer compositions are continually developed and characterized in terms of mechanical behavior. In this context, the countless possibilities to compound different polymers and additives in order to obtain new polymer compositions are an ideal prerequisite for integrating and establishing the computer implemented method as a standard tool.

Polymer composition:

In order to exploit the prediction force of the method, it is preferred when the test polymer composition differs from each of the training polymer compositions in at least one feature, *e.g.* in the content of one or more components or in the presence of one or more components. Nevertheless, in one embodiment of the method, each of the training polymer compositions and the test polymer composition is a polyolefin composition, preferably a polyolefin composition which comprises from 50.0 to 100.0 wt.-%, more preferably from 60.0 to 99.8 wt.-%, most preferably from 70.0 to 95.0 wt.-% of a polyolefin. The polyolefin may be selected from a virgin polyolefin or a recycled polyolefin or a blend of those. In general, a blend denotes a mixture of two or more components, wherein at least one of the components is polymeric. Such blends can be prepared by mixing the two or more components according to established mixing procedures.

A virgin polyolefin is a material that is newly produced, in particular from monomeric units such as alpha olefins. The term "virgin" may, furthermore, apply to a material which has not yet completed a first use cycle and/or has not yet been recycled.

In contrast to that, the recycled polyolefin is preferably recovered from waste plastic material derived from post-consumer and/or post-industrial waste. Post-consumer waste refers to objects having completed at least a first use cycle (or life cycle), *i.e.* having already served their first purpose; while industrial waste refers to manufacturing scrap, which does not normally reach a consumer. Further preferably, the recycled polyolefin comprises at least 88 wt.-%, more preferably at least 92 wt.-%, most preferably at least 96 wt.%, of polyolefins.

In terms of analytic methods, virgin polyolefins and recycled polyolefins may be differentiated based on the absence or presence of contaminants, e.g. limonene and/or fatty acids and/or paper and/or wood.

To be more specific, each of the training polymer compositions and the test polymer composition may be a polyolefin composition and/or a composition which comprises from 50.0 to 100.0 wt.-%, more preferably from 60.0 to 99.8 wt.-%, most preferably from 70.0 to 95.0 wt.-% of a polyolefin, wherein the polyolefin is, in particular, selected from the group consisting of recycled propylene; recycled polyethylene; virgin polyolefin, e.g. virgin polypropylene and/or virgin polyethylene; and blends thereof. Blends may have a weight ratio of polypropylene : polyethylene from 20 : 80 to 80 : 20.

Virgin polyolefins, which may also be contained in the composition that is used as training polymer composition and test polymer composition, preferably comprise at least one homopolymer and/or and least one copolymer. The homopolymer may be a polypropylene homopolymer and/or a polyethylene homopolymer. The copolymer may be selected from the group consisting of copolymers of ethylene and an alpha-olefin comonomer having 3-12 carbon atoms, preferably 3-6 carbon atoms, and copolymers of propylene and an alpha-olefin comonomer having 4-12 carbon atoms, preferably 4-6 carbon atoms.

It should be understood that the polyolefin compositions which are used as training polymer compositions and test polymer composition may comprise one or more further components apart from the polyolefin described above. The further component(s) may be contained in an amount of 45 wt.-% or less, preferably in an amount of 41 wt.-% or less, more preferably in an amount of 25 wt.-% or less.

Impact modifier

According to another option, the test polymer composition and/or the training polymer compositions is/are polyolefin compositions as described above, further comprising at least one or more impact modifier, wherein the total content of the impact modifier is preferably from 0.1 to 50 wt.-%, more preferably from 2.0 to 45 wt.-%, based on the total weight of the test polymer composition and/or the training polymer compositions.

The impact modifier may be a heterophasic polypropylene. The total content of the heterophasic polypropylene is preferably from 0.1 to 50 wt.-%, more preferably from 2.0 to 45 wt.-%, based on the total weight of the test polymer composition and/or the training polymer compositions. The term heterophasic polypropylene denotes a propylene-based copolymer (i.e. a heterophasic propylene copolymer, HECO) with a crystalline matrix phase, which can be a propylene homopolymer or a random copolymer of propylene and at least one alpha-olefin comonomer, and an elastomeric phase dispersed therein. In case of a random heterophasic propylene copolymer (RAHECO), the crystalline matrix phase is a random copolymer of propylene and at least one alpha-olefin comonomer.

The impact modifier may, alternatively or additionally, be a plastomer and/or an elastomer and/or a copolymer of ethylene and methyl acrylate. Suitable elastomers may be ethylene/propylene copolymers with different ethylene/propylene ratio (C2/C3 or C3/C2 elastomers), ethylene/butene copolymers (C2/C4 elastomers), ethylene/octene copolymers (C2/C8 elastomers), grafted ethylene elastomers (such as maleic anhydride grafted ethylene elastomers) or C2/C3 and C2/C4 block copolymers, in particular an ethylene based 1-octene elastomer. Commercially available C2C8 impact modifiers are marketed under the trademarks Engage, Queo, Exact, Tafmer, Infuse and the like. As the maleic anhydride grafted ethylene elastomer commercially available impact modifiers marketed under the trademark name Fusabond may be used.

The impact modifier preferably has an $MFR_2$ (2.16 kg, 190 °C) of 1 to 20 g/10 min, more preferably 1 to 10 g/10 min. The impact modifier further preferably has an $MFR_2$ (2.16 kg, 230 °C) of 2 to 12 g/10 min.

Additive

Alternatively or additionally, the test polymer composition and/or the training polymer compositions is/are polyolefin compositions as described above, further comprising at least one additive. The total content of the additive is preferably from 0.01 to 1 wt.-%, more preferably from 0.05 to 0.5 wt.-%, based on the total weight of the test polymer composition and/or the training polymer compositions. Suitable additives are well known in the art and can, for example, be selected from the group consisting of antioxidants, anti-acids, antiblocking agents, UV protecting agents, nucleating agents and antistatic agents and combinations thereof, with antioxidants being the most preferred in the context of the present invention.

Examples of antioxidants which are commonly used in the art, are sterically hindered phenols (such as CAS No. 6683-19-8, also sold as Irganox 1010 FF™ by BASF, or Irganox 225™ by BASF), phosphorous based antioxidants (such as CAS No. 31570- 04-4, also sold as Hostanox PAR 24 (FF)™ by Clariant, or Irgafos 168 (FF)TM by BASF), sulphur based antioxidants (such as CAS No. 693- 36-7, sold as Irganox PS- 802 FL™ by BASF), nitrogen-based antioxidants (such as 4,4'- bis(l,l' -dimethyl- benzyl)diphenylamine), or antioxidant blends.

Anti-acids (so-called acid scavengers) are also commonly known in the art. Examples are calcium stearates, sodium stearates, zinc stearates, magnesium and zinc oxides, synthetic hydrotalcite (e.g. SHT, CAS-No. 11097-59-9), lactates and lactylates, as well as calcium stearate (CAS No. 1592-23-0) and zinc stearate (CAS No. 557-05-1);

Common antiblocking agents are natural silica such as diatomaceous earth (such as CAS No. 60676-86-0 (SuperfFloss™), CAS-No. 60676-86-0 (SuperFloss E™), or CAS-No. 60676-86-0 (Celite 499™)), synthetic silica (such as CAS-No. 7631-86-9, CAS-No. 7631-86-9, CAS-No. 7631-86-9, CAS-No. 7631-86-9, CAS-No. 7631-86-9, CAS-No. 7631-86-9, CAS-No. 112926-00-8, CAS-No. 7631-86-9, or CAS-No. 7631-86-9), silicates (such as aluminum silicate (Kaolin) CAS-no. 1318-74-7, sodium aluminum silicate CAS-No. 1344-00-9, calcined kaolin CAS-No. 92704-41-1, aluminum silicate CAS-No. 1327-36-2, or calcium silicate CAS-No. 1344-95-2), synthetic zeolites (such as sodium calcium aluminosilicate hydrate CAS-No. 1344-01-0, CAS-No. 1344-01-0, or sodium calcium aluminosilicate, hydrate CAS-No. 1344-01-0).

UV protecting agents are, for example, Bis-(2,2,6,6-tetramethyl-4-piperidyl)-sebacate (CAS-No. 52829-07-9, Tinuvin 770); 2-hydroxy-4-octyloxy-benzophenone (CAS-No. 1843-05-6, Chimassorb 81).

Nucleating agents like sodium benzoate (CAS No. 532-32-1); 1,3:2,4-bis(3,4-dimethylbenzylidene)sorbitol (CAS 135861-56-2, Millad 3988).

Suitable antistatic agents are, for example, glycerol esters (CAS No. 97593-29-8) or ethoxylated amines (CAS No. 71786-60-2 or 61791-31-9) or ethoxylated amides (CAS No. 204-393-1).

It is to be understood that further additives may also be included in the polyolefin composition and that the sum of all ingredients always adds up to 100 wt.-% in each of the embodiments described herein.

Training Phase - Input

With reference to the computer implemented method, it is preferred that the training polymer compositions include compositions differing from each other in the types and/or amounts of the polyolefin and/or the optional further component and/or in the conditions applied in the manufacturing process. In this way, the training polymer compositions are representative of the whole population that is relevant and from which the test polymer composition is taken. Compositions differing from each other in the type and amount of impact modifier are, for example, particularly preferred candidates for training polymer compositions. It is believed that such compositions cover a broad range of impact strengths and are sufficiently distinct from each other so that the trained machine learning algorithm provides robust and accurate outputs for "unseen" compositions with arbitrary impact strength.

Furthermore, in order to be at least fairly representative of the whole population for which predictions of the DBTT should be made, the training in the first step of the method according to the present invention is preferably carried out with data sets extracted from impact curves of at least 15, more preferably at least 30, in particular at least 40 different training polymer compositions and/or the training in the first step of the method is preferably carried out with data sets extracted from at least 50, more preferably at least 100, in particular at least 1000 impact curves of training polymer compositions.

Below the threshold value of 15 for the minimum number of training polymers, reliability of the prediction and the method suffers. Besides that, it is assumed that the benefit of using data sets extracted from impact curves of more than 500, in particular more than 250, different training polymer compositions, will not contribute to a meaningful benefit in the accuracy of the method. On the other hand, deviation of the recommended minimum number of impact curves from the recommended minimum number for the different training polymer compositions is due to the fact that it is preferable to collect impact curves of one and the same training polymer composition at different measurement conditions. For example, the impact curves of the training polymer compositions in the first step of the method described above have preferably been obtained in a temperature range from -70 °C to +60 °C.

Technically speaking, the impact curves of the training polymer compositions, as well as of at least one impact curve of the test polymer composition, may be obtained by instrumented experimentation, preferably in a temperature range from -70 °C to +60 °C. The impact curves are preferably force-displacement curves. The force-displacement curves may be obtained according to an impact test or a curve derived thereof. The impact test itself may be a standardized test and may be selected from the tests consisting of a Charpy impact test according to ISO 179-2:2020, an Izod impact test according to ASTM D256 or ISO180; and a puncture impact test according to ISO 6603-2:2002-04 (hereinafter also instrumented puncture test, IPT).

Prediction Phase - Input

It has advantages if the input in the second step of the method (prediction phase) is comparable to the input that has been provided in the first step (training phase). This can be achieved in several ways. For example, the impact curve of the test polymer composition has preferably been obtained in the same temperature range as the impact curves of the training polymer compositions, for example in a range from 70 °C to +60 °C. Furthermore, the same instrumentation or measurement equipment has preferably been used to obtain the impact curves of the test polymer composition and the training polymer composition. Also, the impact curves of the test polymer composition are preferably force-displacement curves recorded according to the same standards mentioned for the training polymer compositions above.

However, and this already implicitly follows from the explanations above, the composition of the test polymer preferably differs from the test polymer compositions. Even the components or the type of components in the test polymer composition are not limited to the components or type of components in the training polymer compositions. If the number and diversity of the training polymer compositions has been appropriately chosen, the trained machine algorithm can successfully be applied to chemically different polymer compositions. In this case, the value of the computer implemented method is further increased since test polymer compositions do not have to be analyzed with respect to the kind of components comprised to decide whether they are eligible. Instead, the data set(s) are extracted from at least one impact curve of the test polymer composition without further experimentation and the trained machine algorithm is fed with these data as an input.

Furthermore, in an embodiment, the method according to the present invention comprises training a machine learning algorithm with training polymer compositions by mapping data sets extracted from impact curves of the training polymer compositions to values indicative of the ductile-to-brittle transition temperatures of the training polymer compositions in a first step; feeding the trained algorithm, as an input, with corresponding data set(s) extracted from 1 to 3 impact curve(s) of the test polymer composition, preferably 1 or 2 impact curve(s) of the test polymer composition in a second step; and receiving, as an output, a value indicative of the ductile-to-brittle transition temperature of the test polymer composition in a third step.

Operating the method as described above, namely with only a limited number of data sets representative of the test polymer composition as an input for the prediction phase, is beneficial. In particular, it allows to minimize experimental effort and testing time that is associated with the prediction of the DBTT of a test polymer composition.

Type of Data Set(s)

The data sets extracted from impact curves of training polymer compositions and the data set(s) extracted from the at least one impact curve of the test polymer composition may comprise or consist of force-displacement data pairs as read from the respective impact curve; or values of curve characteristics derived from the impact curve, wherein the curve characteristics are preferably selected from the group consisting of: the maximum force; displacement at maximum force; area under curve representative of energy; highest deflection; number of local force maxima; average force; average deflection; median force; median deflection; skew of the distribution of force values; kurtosis of the distribution of force values; skew of the distribution of energy values; kurtosis of the distribution of energy values; standard deviation of force values; variance of force values; and combinations thereof.

In the case in which each of the data sets extracted from impact curves of training polymer compositions and/or the data set(s) extracted from the at least one impact curve of the test polymer composition comprise(s) or consist(s) of curve characteristics derived from the impact curve, the data set(s) may comprise more than one value per curve characteristic, each value being derived from a different segment of the impact curve. For example, there may be a first value for the average force derived from a first segment of the impact curve, a second value for the average force derived from a second segment of the impact curve and a third value for the average force derived from a third segment of the impact curve.

The process by which the curve characteristics are obtained, *i.e.* extracted from the impact curves of training polymer compositions and/or the at least one impact curve of the test polymer composition is denoted feature engineering. The details of the feature engineering process will be explained in more detail below.

Computational Implementation

In an embodiment, the machine learning algorithm is based on random forest regression (RFR) or an artificial neural network, in particular a convolutional neural network (CNN).

In terms of the computational realization, the RFR and the CNN may be implemented using open-source software. For example, the RFR may be implemented with Scikit-learn, a software which is based on Python programming language. The CNN may be implemented based on a 1D AlexNet which is connected to a fully convolutional neural network regression using the Pytorch libraries.

Random Forest Regression

Mechanistically, the RFR is a decision tree-based machine learning algorithm. Decision trees may be understood as flowchart-like diagrams used to compute an outcome. Each branch of the tree represents a possible decision. The tree is structured to show how and why one choice may lead to the next, with branches indicating that each option is mutually exclusive. Decision trees comprise a root node, leaf nodes and branches. The root node is the starting point of the tree. Both root and leaf nodes contain criteria to be applied. Branches are arrows connecting nodes, showing the flow from question to answer. A specific feature of the RFR is that multiple decision trees are randomly created and the results of the single trees are combined to generate an output.

Feature Engineering

Deriving values of curve characteristics from the impact curves may be considered an optional feature engineering step with helps to further improve the training result.

As mentioned before, the derived curve characteristics are preferably curve characteristics selected from the group consisting of the maximum force; displacement at maximum force; area under curve representative of energy; highest deflection; number of local force maxima; average force; average deflection; median force; median deflection; skew of the distribution of force values; kurtosis of the distribution of force values; skew of the distribution of energy values; kurtosis of the distribution of energy values; standard deviation of force values; variance of force values; and combinations thereof. Such data sets can appropriately be processed and handled by a RFR based machine learning algorithm and assist in achieving an excellent training result.

Before deriving values of curve characteristics from the impact curves of the training polymer compositions and the at least one impact curve of the test polymer composition, it may be helpful to divide the respective impact curve(s) into different segments. These segments may be derived based on physics or mechanical response considerations and may represent different domains of a response to an impact. In the case of a force-displacement curve, the segments may, for example, be determined by a two straight connection lines between the peak force and the force at the beginning and the end of the experiment and those points on the curve with the largest orthogonal distance to these connection lines. Values of curve characteristics may then be determined for each segment. Accordingly, the data sets extracted from impact curves of training polymer compositions and the at least one impact curve of the test polymer composition preferably comprise more than one value per curve characteristic, each value being derived from a different segment of the impact curve.

Size of Data Set(s) and Cluster Analysis

In an embodiment, each of the data sets extracted from impact curves of training polymer compositions and/or the data set(s) extracted from the at least one impact curve of the test polymer composition comprises at least 30, more preferably at least 50, most preferably at least 70 single data pairs or values. As a rule of thumb this will provide the necessary level of detail.

However, the data sets can also become too large, resulting in unfavorably long computation times. To avoid this or that the above described feature engineering step introduces a too high level of complexity, the training in the first step of the method may further comprise the application of a technique to reduce the size of the data sets, such as cluster analysis. In other words, each of the data sets extracted from impact curves of training polymer compositions has preferably been reduced by a pattern recognition technique, preferably by hierarchical cluster analysis or principal component analysis (PCA) before the training phase. This technique allows assessing whether the size of the data set that is used during training is necessary or whether it can be reduced at a small expense. Put simply, this may be done by reducing the force-displacement data pairs read from or the number of curve characteristics that are derived from the impact curves for the training step to a desirable extent. If the reduction does not result in a significant decrease of the generalization performance of the method, the size of the data set that is used during training may be adjusted accordingly. The same reduction can also be applied to the data set(s) derived from the at least one impact curve of the test polymer composition before the prediction phase.

Artificial Neural Network

The term "artificial neural network" refers to an algorithm that mimics the operation of the human brain. For processing, it comprises artificial neurons arranged in input layers, output layers and hidden layers. In the hidden layers, each neuron receives input signals from other neurons and uses this input for the further computation. The connections between the neurons have weights, the values of which represent the stored knowledge of the network. Learning is the process of adjusting the weights so that the training data can be reproduced as accurately as possible.

A CNN, as mentioned above as a preferred embodiment for the machine learning algorithm, is a specialized type of an artificial neural network. It performs a mathematical operation called "convolution" in at least one of the layers. In a formula, a convolution may be represented by the following equation (1)

$$s(t) = (x * w)(t) \quad (1),$$

with the first argument (the function x in the formula above) to the convolution being the input, the second argument (the function w in the formula above) being the kernel and the result (the function $s$ in the formula above) being the feature map. In machine learning applications, the input is usually a multidimensional array of data. Likewise, the kernel is a multidimensional array of parameters. Both arrays are adapted by the learning algorithm.

In terms of the present computer implemented invention, a machine learning algorithm based on CNN is preferably used when the data sets extracted from impact curves of the training polymer compositions are a time dependent deflection signal and/or a time-dependent force signal. Such signals have a grid-like structure. In other words, these signals can be considered 1-D grids of measurement values of a sample at (regular) time intervals. Such a grid-like input is particularly suitable for training a CNN.

Hyperparameter Optimization and Cross-Validation

At least one additional step selected from hyperparameter optimization and cross-validation may be included in the method irrespective of the model (*e.g.* RFR or CNN) on which the machine learning algorithm is based. In this case, training step a) comprises a first training step and a second training step and the method comprises at least one additional step selected from cross-validation and hyperparameter optimization, the at least one additional step being preferably performed after the first training step and before the second training step. Hyperparameter optimization is a means of fine-tuning the machine learning algorithm, in particular to achieve a better performance of the method for predicting the DBTT. The initial values of the hyperparameters, *i.e.* the internal variables of the algorithm, may not result in the best possible performance because they may be estimated using systematic and random searches, or heuristics at the beginning of the training phase.

The cross-validation step may be a single cross-validation step or a *k*-fold cross-validation step. A *k*-fold cross-validation step can be carried out with *k* preferably being a number from 1 to 20, in particular 1 to 10.

For a single cross-validation step (*k* = 1), a randomly selected portion of the data sets extracted from impact curves of the training polymer compositions may be withheld during the first training step. This withheld portion, which may comprise from 10 to 40% of the data sets and may be denoted as the "validation set", can be fed into the algorithm once the first training step has been completed. The extent of accuracy to which the DBTTs are predicted for the validation set, may be regarded as a measure of the effectiveness of the training phase and the quality of the hyperparameters. Furthermore the hyperparameters may be adapted. Modules within the computational software that is used may take over the outlined optimization procedure of the hyperparameters. As a result, optimized hyperparameters may be determined by grid-search in a nested cross validation loop. At this point, the second training step, *i.e.* a mapping of data sets extracted from impact curves of the training polymer compositions to values indicative of ductile-to-brittle transition temperatures of the training polymer compositions, may be performed based on the optimized hyperparameters.

In a 5-fold cross-validation step (*k* = 5), the data sets extracted from the impact curves of the training polymer compositions are split into 5 partitions of comparable size. Four partitions are used for training, while one is used as a validation set. This process is repeated 5 times, with a different partition used as the validation set each time. For evaluation purposes, the average or median of all folds can be calculated. Such an average or median provides a better prediction, however, a *k*-fold cross-validation also increases training cost in terms of computation time.

In any case, the assignment of the data sets to a training ensemble and a validation ensemble is a critical step in the optional cross-validation. Cross-validation is only reliable when the assignment is in line with the characteristics of the whole dataset. In other words, data of a particular species or class may not be heavily underrepresented in either of the ensembles. Hence, it may be advisable to supervise whether the technique that is applied in order to obtain a randomly selected portion of the whole data set, yields acceptable results.

DBTT

The value(s) indicative of the DBTT may be an absolute temperature or a temperature difference. In the scenario in which the value is an absolute temperature, the value may be the ductile-to-brittle-temperature itself. Otherwise, the value may be an indirect measure of the DBTT, for example in the form of a temperature difference, particularly a temperature difference according to equation (2) below

$$\Delta T = DBTT - T \qquad (2),$$

wherein T is a reference temperature or a temperature at which the impact curve of the test polymer composition has been obtained. This allows predictions to be a measure of the relative difference to a temperature of interest. Moreover, the sign of the difference will indicate whether the deviation is in the direction of increasing temperature or decreasing temperature on the temperature scale.

Controlling Step

The output of the computer implemented method according to the present invention may be used to make a decision on the further fate of the test polymer composition. For example, if the test polymer composition is a candidate composition which is suspected to satisfy the demands of a target application, the value indicative of the DBTT as output by the present method may bring clarity. The output value may further be used to determine whether the test polymer composition is taken to the next research stage. More generally, the value indicative of the DBTT may be used for controlling a process in a development of application-tailored polymer composition.

Means for Storage and Execution of the Computer Implemented Method

According to another aspect, a non-transitory computer readable storage medium for tangibly storing computer program instructions capable of being executed by a processor is provided, the computer program instructions defining the steps of the method as described above.

However, the present invention also includes further means for storage and execution of the computer implemented method as described above. For example, the invention extends to methods and apparatuses which perform the method described above, including data processing systems which perform the method and computer readable media containing instructions which when executed on data processing systems cause the systems to perform the method.

Non-volatile memory can be a local device coupled directly to the rest of the components in the data processing system. A non-volatile memory that is remote from the system, such as a network storage device coupled to the data processing system through a network interface such as a modem or Ethernet interface, can also be used.

In the present disclosure, some functions and operations are described as being performed by or caused by software code to simplify description. However, such expressions are also used to specify that the functions result from execution of the code/instructions by a processor, such as a microprocessor.

Alternatively, or in combination, the functions and operations as described here can be implemented using special purpose circuitry, with or without software instructions, such as using Application-Specific Integrated Circuit (ASIC) or Field-Programmable Gate Array (FPGA). Embodiments can be implemented using hardwired circuitry without software instructions, or in combination with software instructions. Thus, the techniques are limited neither to any specific combination of hardware circuitry and software, nor to any particular source for the instructions executed by the data processing system.

While one embodiment can be implemented in fully functioning computers and computer systems, various embodiments are capable of being distributed as a computing product in a variety of forms and are capable of being applied regardless of the particular type of machine or computer-readable media used to actually effect the distribution.

At least some aspects disclosed can be embodied, at least in part, in software. That is, the techniques may be carried out in a computer system or other data processing system in response to its processor, such as a microprocessor, executing sequences of instructions contained in a memory, such as ROM, volatile RAM, non-volatile memory, cache or a remote storage device.

Routines executed to implement the embodiments may be implemented as part of an operating system or a specific application, component, program, object, module or sequence of instructions referred to as "computer programs." The computer programs typically include one or more instructions set at various times in various memory and storage devices in a computer, and that, when read and executed by one or more processors in a computer, cause the computer to perform operations necessary to execute elements involving the various aspects.

A machine-readable medium can be used to store software and data which when executed by a data processing system causes the system to perform various methods. The executable software and data may be stored in various places including for example ROM, volatile RAM, non-volatile memory and/or cache. Portions of this software and/or data may be stored in any one of these storage devices. Further, the data and instructions can be obtained from centralized servers or peer to peer networks. Different portions of the data and instructions can be obtained from different centralized servers and/or peer to peer networks at different times and in different communication sessions or in a same communication session. The data and instructions can be obtained in entirety prior to the execution of the applications. Alternatively, portions of the data and instructions can be obtained dynamically, just in time, when needed for execution. Thus, it is not required that the data and instructions be on a machine-readable medium in entirety at a particular instance of time.

Examples of computer-readable media include but are not limited to non-transitory, recordable, and non-recordable type media such as volatile and non-volatile memory devices, Read Only Memory (ROM), Random Access Memory (RAM), flash memory devices, floppy and other removable disks, magnetic disk storage media, optical storage media (e.g., Compact Disk Read-Only Memory (CD ROM), Digital Versatile Disks (DVDs), etc.), among others. The computer-readable media may store the instructions.

The instructions may also be embodied in digital and analog communication links for electrical, optical, acoustic or other forms of propagated signals, such as carrier waves, infrared signals, digital signals, etc. However, propagated signals, such as carrier waves, infrared signals, digital signals, etc. are not tangible machine readable medium and are not configured to store instructions.

In general, a machine-readable medium includes any mechanism that provides (i.e., stores and/or transmits) information in a form accessible by a machine (e.g., a computer, network device, personal digital assistant, manufacturing tool, any device with a set of one or more processors, etc.).

In various embodiments, hardwired circuitry may be used in combination with software instructions to implement the techniques. Thus, the techniques are neither limited to any specific combination of hardware circuitry and software nor to any source for the instructions executed by the data processing system.

<u>Use</u>

According to yet another aspect, the use of impact curves and values indicative of ductile-to-brittle transition temperatures of polymer compositions for training a machine learning algorithm is disclosed. Use of impact curves is advantageous since these curves can easily be obtained and reproduced with standard equipment. Furthermore, no particular safety concerns are associated with impact measurement methods so that no special precautionary measures have to be taken. Furthermore, a value indicative of the ductile-to-brittle transition temperature of a polymer composition is accessible from a series of experimentally obtained impact curves at different temperatures.

**Brief Description of the Drawings**

Embodiments of the present invention are illustrated by way of example and are not limited by the figures of the accompanying drawings in which identical references represent similar elements.

Fig. 1 shows an exemplary flow diagram illustrating the method steps of the method according to the present invention when carried out with a RFR based machine learning algorithm;

Fig. 2 shows a force-displacement curve of a polymer composition which is divided into six different segments for the feature engineering step;

Figs. 3a and 3b visualize the prediction performance of a method that employs a RFR based machine learning algorithm for PP-based test polymer compositions and for PE/PP-based test polymer compositions, respectively;

Fig. 4 shows an exemplary flow diagram illustrating the method steps of the method according to the present invention when carried out with a CNN based machine learning algorithm;

Fig. 5 shows an exemplary flow diagram illustrating a part of the method according to the present invention when carried out with a CNN based machine learning algorithm alongside the main input parameters to define an AlexNet 1D model with an open source software;

Fig. 6 shows an exemplary flow diagram illustrating another part of the method according to the present invention when carried out with a CNN based machine learning algorithm alongside the main input parameters to define the FCN (fully convolutional network) with an open source software;

Fig. 7 illustrates a cross-validation procedure which may be adopted in a CNN or RFR based machine learning algorithm using five different dataset splits;

Figs. 8a and 8b visualize the prediction performance of a method that employs a CNN based machine learning algorithm for PE-based test polymer compositions and for PE/PP-based test polymer compositions separately.

**Detailed Description of the Drawings and Examples**

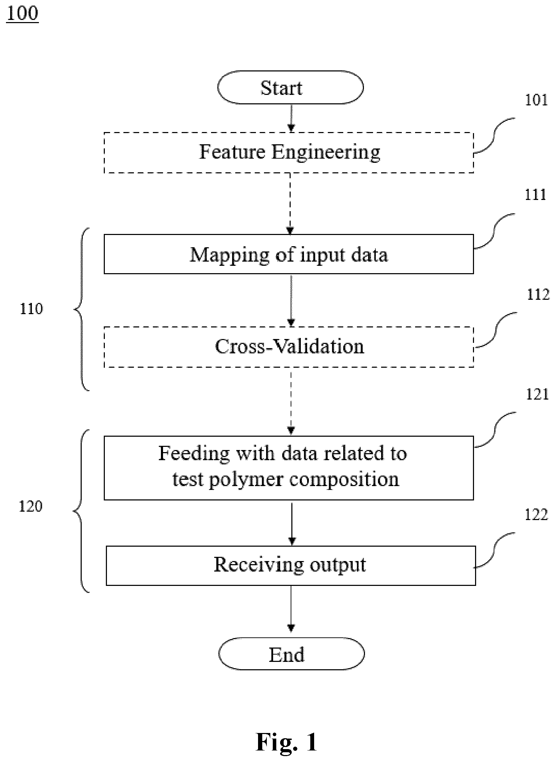

Fig. 1 shows an exemplary flow diagram illustrating the method steps of the method 100 according to the present invention. Optional steps of the method 100 are presented in dashed lines.

In an optional step 101, feature engineering is performed. During feature engineering the size of the input data is reduced. For example, force-displacement curves may be divided into segments and specific curve characteristics may be extracted from each of the segments. The sum of the extracted curve characteristics is less than the sum of single data pairs constituting the force-displacement curve.

In step 111, mapping of the input data is performed to train the algorithm. For example, the data sets extracted from the impact curves of the training polymer compositions may be mapped to the values indicative of the ductile-to-brittle transition temperatures that have been measured or are otherwise known for the training polymer compositions.

In step 112 an optional cross-validation takes place. During the cross-validation the initial values selected for the hyperparameters may also be adapted and optimized.

Together steps 111 and 112 of the method constitute the training phase 110 of the model.

The prediction phase 120 comprises further steps of the method: In step 121, the algorithm is fed with at least one data set related to the test polymer composition. In particular, data set(s) extracted from at least one impact curve of the test polymer composition may be inputted into the algorithm. These data sets may be obtained in a similar fashion as the data sets that have been inputted in the training phase. Last but not least, in outputting step 122, the method returns as an output a value indicative of the DBTT of the test polymer composition.

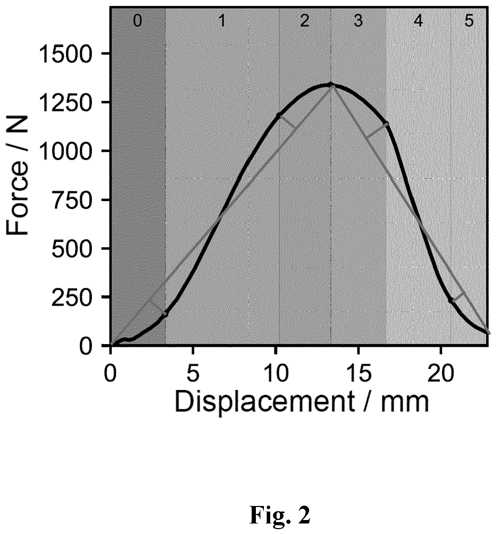

Fig. 2 shows a force-displacement curve of a polymer composition which is divided into six different segments for the feature engineering step. Each of the segments is related to different stage of deformation and eventual failure of the polymer composition during the experiment (e.g. initial elastic loading, initiation of plastic deformation, plastic yielding, failure, etc). As indicated in the graph, the division into the segments has been made based on a specific mathematical definition (involving the orthogonal distance to a first connection line between initial force and peak force and a second connection line between peak force and force at the end of the experiment).

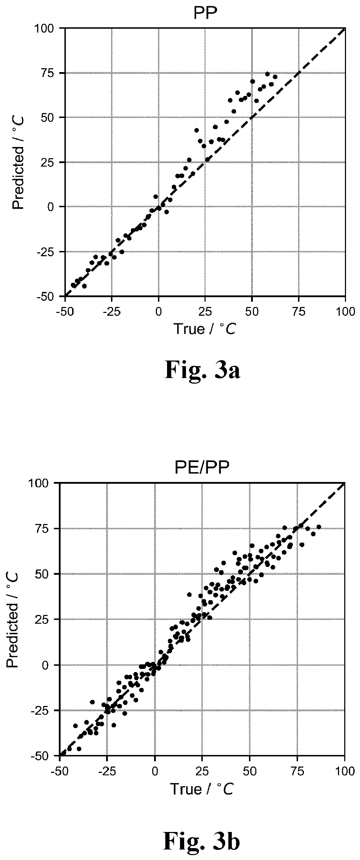

Figs. 3a and 3b contain correlation plots which demonstrate the very good prediction performance of the inventive method employing a RFR based machine learning algorithm based PP-based test polymer compositions (Fig. 3a) and PE/PP-based test polymer compositions (Fig. 3b). Values indicative of the "true" DBTTs (i.e. experimentally obtained DBTTs) have been plotted on this x-axis whereas the predicted values indicative of the DBTTs have been plotted on the y-axis.

As usual for correlation plots, an ideal prediction would require that all points come to lie exactly on the diagonal line. In the current case, the points in Figs. 3a and 3b follow the diagonal line fairly well but are slightly scattered to both sides. Yet, the deviation from the diagonal line is, overall, small. Hence, the correlation plots in Figs. 3a and 3b indicate a very good prediction performance of the trained machine learning algorithm.

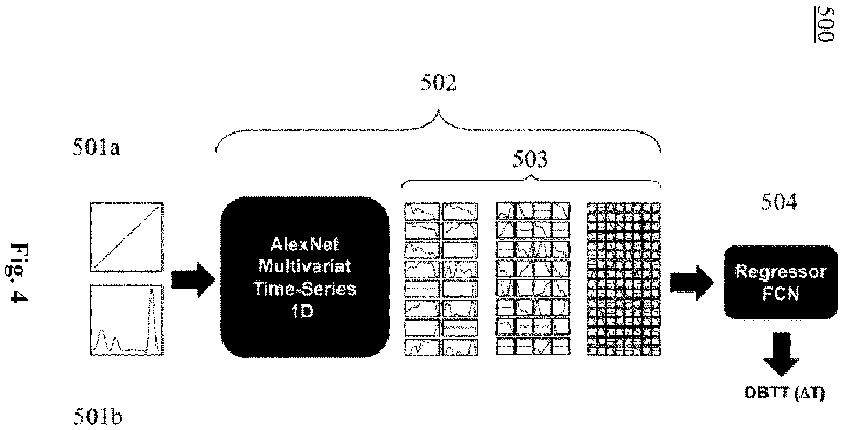

Fig. 4 shows an exemplary flow diagram illustrating the method steps of the method according to the present invention when carried out with a CNN based machine learning algorithm.

In a first step, a time dependent deflection signal 501a and a time dependent force signal 501b, which have been derived from an experimental impact curve of a polymer composition, are fed into a trained CNN based algorithm based on a 1D AlexNet 502.

The CNN performs several convolutions in parallel to produce a set of linear activations. Each linear activation is run through a nonlinear activation function, such as the rectified linear activation function (ReLU). Furthermore, three max pooling operations are used to modify the output of the layer further. A pooling function replaces the output of the net at a certain location with a summary statistic of the nearby outputs. The max pooling operations, illustrated as 503, report the maximum output within a rectangular neighborhood and manage to reduce the size of the dataset that is handled.

Feeding the obtained dataset into the fully convolutional neural network regressor, FCN, 504 allows to obtain the value indicative of the DBTT of the polymer composition.

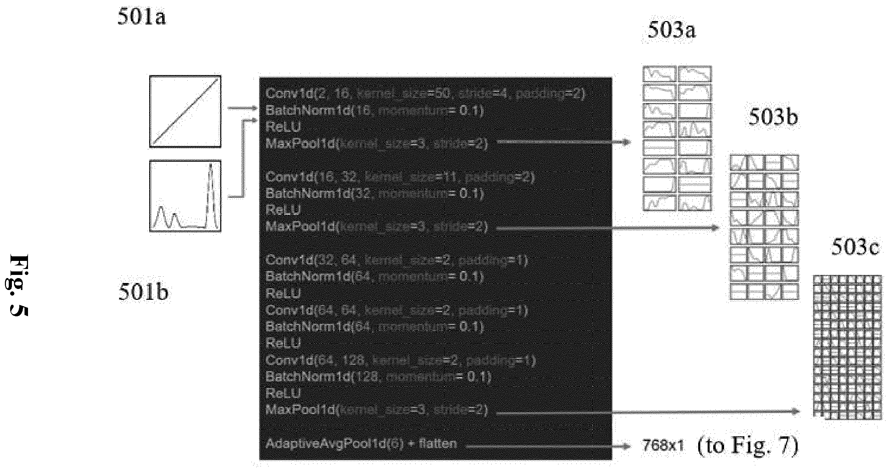

Fig. 5 shows the main input parameters for defining the 1D AlexNet based machine learning algorithm of fig. 4.

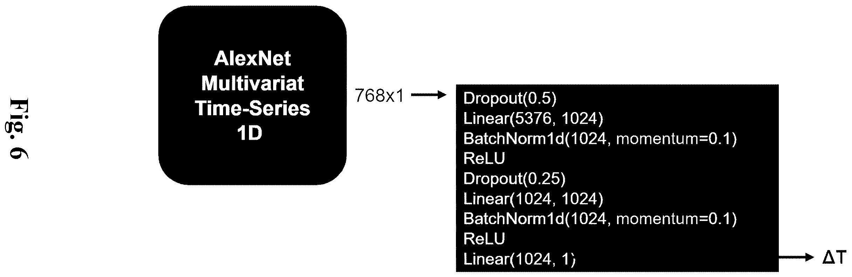

Fig. 6 shows the main input parameters for defining the fully convolutional neural network regressor already shown in fig. 4.

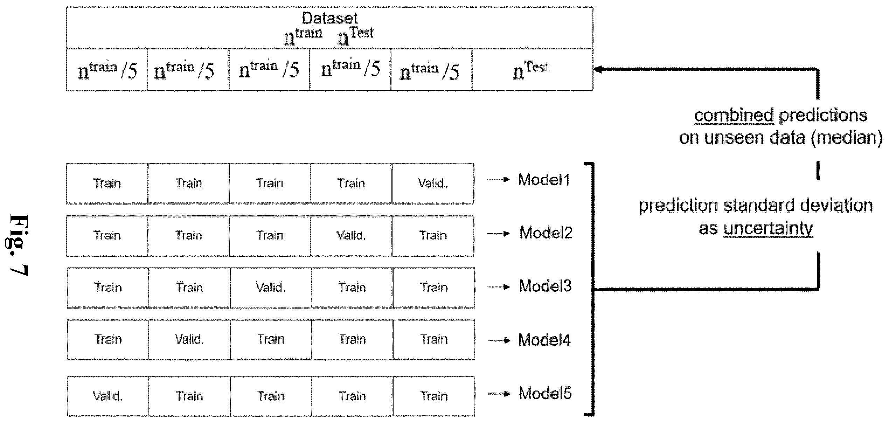

Fig. 7 illustrates a cross-validation procedure which may be adopted in a CNN or RFR based machine learning algorithm using five different dataset splits. A different portion of the complete dataset is treated as the training dataset (Train) and the test dataset (Valid) each time the algorithm is run. Five separate trained algorithms are obtained in parallel in this way (Model 1-Model5). The median of the outputs of these five models is taken as the final value indicative of the DBTT.

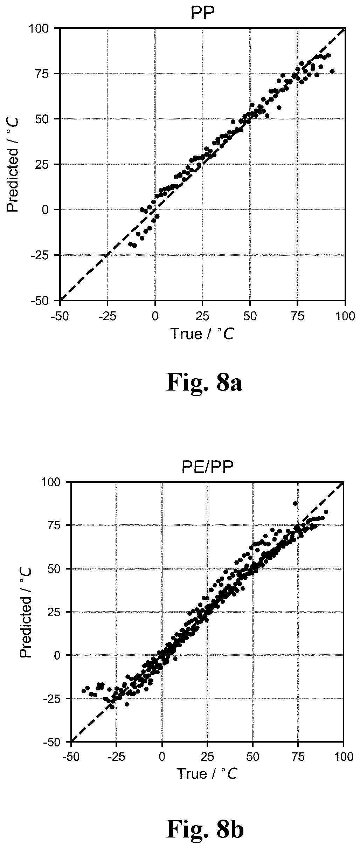

Figs. 8a and 8b are analogous to Figs. 3a and 3b. Just like figs. 3a and 3b, figs. 9a and 9b contain correlation plots. This time, the correlation plots demonstrate the very good prediction performance of the inventive method employing a CNN based machine learning algorithm based on PP-based test polymer compositions (Fig. 8a) and PE/PP-based test polymer compositions (Fig. 8b). Values indicative of the "true" (i.e. experimentally obtained) DBTTs have been plotted on this x-axis whereas the predicted values indicative of the DBTTs have been plotted on the y-axis.

**Examples**

The nature of the present invention will become more clearly apparent in view of the accompanying examples. The examples should, however, in no way limit the scope of the invention.

Example 1

A RFR based machine learning algorithm has been implemented in scikit-learn. It has been trained and tested in the following way:
164 different polymer compositions have been provided. Each of these polymer compositions contained a polyolefin (either PE, PP or both) and an impact modifier. The impact modifier was either a heterophasic propylene copolymer (HECO), an elastomeric or plastomeric impact modifier (MOD), or another kind of impact modifier (PO). The polymer compositions have been measured in an instrumented puncture test according to ISO 6603-2:2002-04. This resulted in 7489 individual force-deflection curves at temperatures between -60°C and 51°C.

The polymer compositions have been categorized based on their chemical constitution. Eight different chemical categories have been identified: PE/PP, PE/PP_MOD, PE/PP_HECO, PE/PP_PO, PE_MOD, PE HECO, PP_MOD and PP_HECO.

Afterwards the polymer compositions and the corresponding force-deflection curves have been split and assigned to a training dataset and a test dataset. The training dataset comprises 60% of the available data and the test dataset comprises the remaining 40% of the available data. Equal distributions of the data to the datasets have been ensured for seven of the eight chemical categories (PE/PP_MOD, PE/PP_HECO, PE/PP_PO, PE_MOD, PE_HECO, PP_MOD and PP_HECO). The force-deflection curves in the category PE/PP, however, have completely been assigned to the test dataset.

Instead of feeding the algorithm with force-displacement data pairs, specific curve characteristics have been extracted from the IPT curves. To that end, feature engineering was applied by splitting the curves into segments, which are delimited by points having the largest orthogonal distance to a first connection line between the initial force and the peak force and a second connection line between the peak force and the force at the end of the experiment (cf. segments 1-6 in Fig. 2). For each of these segments, values for a variety of curve characteristics have been collected, for example for the following characteristics:

- the maximum force;
- displacement at maximum force;
- area under curve representative of energy;
- highest deflection;
- number of local force maxima;
- average force;
- average deflection;
- median force;
- median deflection;
- skew of the distribution of force values;
- kurtosis of the distribution of force values;
- skew of the distribution of energy values;
- kurtosis of the distribution of energy values;
- standard deviation of force values; and
- variance of force values.

Values for these curve characteristics were also extracted for the whole time series. In total, 94 values for curve characteristics have been identified for each force-displacement curve (feature set "ALL"). Additionally, Hierarchical Cluster Analysis has been employed to reduce the number of curve characteristics. This resulted in a reduced feature set "SEL" including 50 values for curve characteristics.

Two separate training runs have been performed with the RFR based machine learning algorithm. One training run was performed with the training dataset based on the feature set ALL and another training run was done with the training dataset based on the feature set SEL. The input predictor variables have been normalized before each of the training runs. Moreover, default values have been chosen as values for the hyperparameters in these trainings.

To reduce the risk of overfitting and to optimize the hyperparameters, a cross-validation step has been carried out in a next step. The following steps have been performed:

- Determining optimal hyperparameters by grid-search in a nested cross validation loop using Python implementation of scikit-learn.
- Fitting models for both, the ALL and the SEL feature set, using the training dataset and the determined hyperparameters.
- Estimate the generalization performance of the trained algorithm for the test dataset.

The results of this procedure are summarized in Table 1 below for the ALL and the SEL feature set:

Table 1: Hyperparameters determined by Grid-search.

| Parameter | Values | ALL | SEL |
|---|---|---|---|
| Number of Trees | [128, 256, 512] | 256 | 256 |
| Max Depth | [20, 25, 30] | 20 | 20 |
| Max Number Features | [40%, 50%, 66%] | 40% | 40% |
| Max Number Samples | [85%, 95%, 99%] | 99%, | 99% |

(continued)

| Parameter | Values | ALL | SEL |
|---|---|---|---|
| Min Samples Split | [2, 4] | 2 | 2 |
| Min Samples Leaf | [1, 2] | 1 | 1 |

After the training phase, the performance of the trained algorithm has been assessed as outlined below.

Evaluation

The performance of the trained algorithm has been evaluated based on PP-based test polymer compositions and PE/PP-based test polymer compositions.

The specific compositions that have been used are listed in table 2 below. Furthermore, table 3 indicates the type of material class that each component belongs to.

For each of the compositions, at least one impact curve has been experimentally obtained by a puncture impact test according to ISO 6603-2:2002-04. Furthermore, for at least some of the compositions, several impact curves have been experimentally obtained by conducting the puncture impact test according to ISO 6603-2:2002-04 at different temperatures in the range from -70 °C to +60 °C. A value indicative of the "true" DBTT (*i.e.* the experimentally obtained DBTT) has been obtained for each experiment.

Table 2: Details of PP-based polymer compositions and PE/PP-based polymer compositions used for evaluation in Example 1.

| Components |
|---|
| Recycled PE/PP + 5 wt.-% Queo 8207 |
| Recycled PE/PP + 20 wt.-% EMA3 |
| Recycled PE/PP + 10 wt.-% RAHECO |
| Recycled PE/PP + 3 wt.-% Infuse 9077 |
| Recycled PP + 20 wt.-% EMA2 |

Table 3: Type of material class that each component belongs to.

| Component | Material class |
|---|---|
| Recycled PP | Polypropylene post-consumer recyclate (commercially available by Borealis AG, with a density according to ISO 1183 of 915 kg/m$^3$ and an MFR$_2$ at 230 °C according to ISO 1133 of 20.0 g/10 min) |
| Recycled PE/PP | Post-consumer recyclate polyblend PE/PP (commercially available by Borealis AG, with a density according to ISO 1183 of 940 kg/m$^3$ and an MFR$_2$ at 230 °C according to ISO 1133 of 5.5 g/10 min) |
| EMA1, EMA2, EMA3 (commercially available by Borealis) | copolymer of ethylene and methyl acrylate |
| Queo 8207, Queo 8201 (commercially available by Borealis) | ethylene based octene-1 elastomer |
| RAHECO (commercially available by Borealis) | random heterophasic polypropylene |
| Infuse 9077 (commercially available by Dow) | ethylene octene (C2C8) block copolymer |

To obtain the value indicative of the DBTT as predicted by the inventive method, a data set extracted from each impact curve of the test polymer compositions has been fed into the trained algorithm.

The experimentally obtained "true" values indicative of the DBTTs of the test polymer compositions have been compared to the predicted values in the plots in Figs. 3a and 3b. Doing so, the difference between the DBTT and the temperature, at which the impact curve of the respective test polymer composition has been obtained, has been assumed to be the value indicative of the DBTT. A very good correlation between true and predicted values has been found in the plots.

Furthermore, the performance has also been quantified numerically: Table 4 below shows the statistics for the cohorts of the test polymer compositions (PP and PE/PP), wherein $n^{train}$ and $n^{Test}$ refer to the number of impact curves used during training phase and test phase (*i.e.* prediction phase), respectively. The mean square error (MSE) and the coefficient of determination ($R^2$) have been used as scoring functions.

Table 4: Quantified performance of RFR based machine learning algorithm for PP-based and PE/PP-based test polymer compositions.

|  | $n^{train}$ | $n^{Test}$ | MSE / °C | $R^2$ |
| --- | --- | --- | --- | --- |
| **PP** | 2177 | 56 | 6.80 | 0.92 |
| **PE/PP** | 2626 | 152 | 5.41 | 0.96 |

Example 2

A CNN based machine learning algorithm based on a 1D AlexNet that is connected to a fully convolutional neural network regressor has been implemented using the Pytorch libraries. It has been trained and tested in the following way:

164 different polymer compositions have been provided. Each of these polymer compositions contained a polyolefin and a further component selected from heterophasic propylene copolymers, impact modifiers and additives. The polymer compositions have been measured in an instrumented Charpy test according to according to ISO 179-2:2020. This resulted in 6205 individual impact curves at temperatures between -40°C and 60°C, from which force-displacement data pairs could be extracted.

Although the polymer compositions are identical to the polymer compositions as used in example 1, the categorization of these data has been carried out differently. Essentially, the polymer compositions have been assigned to three chemical categories: PE (including PE_MOD and PE_HECO from example 1), PP (including PP_MOD and PP_HECO from example 1) and PE/PP (including PE/PP, PE/PP_MOD, PE/PP_HECO and PE/PP_PO from example 1).

The dataset composed of the force-displacement data pairs belonging to 164 different polymer compositions has been split for training and cross-validation purposes. In contrast to the dataset split in example 1, five different dataset splits have been created in the current example. Equal distributions of the data to the datasets have been ensured for each of the three chemical categories in each splitting operation. In nested cross-validations, five separate trained algorithms with optimized hyperparameters have been obtained. The median of the outputs received from the five algorithms is the final prediction value (see illustration in fig. 7). The associated standard deviation is used as a measure of the prediction uncertainty.

After the training phase, the performance of the trained algorithm has, similar as in example 1, been assessed as outlined below.

Evaluation

The performance of the trained algorithm has been evaluated based on PP-based test polymer compositions and PE/PP-based test polymer compositions. The specific compositions that have been used are listed in table 5.

Table 5: Details of PP-based polymer compositions and PE/PP-based polymer compositions used for evaluation in Example 2.

| Components |
| --- |
| Recycled PP + 10 wt.-% EMA1 |
| Recycled PE/PP + 10 wt.-% Queo 8207 |
| Recycled PE/PP + 20 wt.-% EMA1 |
| Recycled PE/PP + 5 wt.-% Queo 8201 |
| Recycled PE/PP + 20 wt.-% Queo 8201 |
| Recycled PP + 20 wt.-% EMA2 |
| Recycled PE/PP + 20 wt.-% EMA2 |
| Recycled PE/PP + 5 wt.-% Infuse 9077 |

Table 3 indicates the type of material class that each component belongs to.

For each of the compositions, at least one impact curve has been experimentally obtained by a Charpy impact test according to ISO 179-2:2020. From the accordingly obtained impact curves force-displacement data pairs have been read and fed into the trained algorithm.

Table 6 below shows the model performance as a function of material category (*i.e.* PE/PP or PP based composition), wherein $n^{train}$ and $n^{Test}$ refer to the number of impact curves used during training phase and test phase (*i.e.* prediction phase), respectively. The mean square error (MSE) and the coefficient of determination ($R^2$) were used as scoring functions.

Table 6: Quantified performance of CNN based machine learning algorithm for PP-based and PE/PP-based test polymer compositions.

| | $n^{train}$ | $n^{Test}$ | MSE / °C | $R^2$ |
| --- | --- | --- | --- | --- |
| **PP** | 1326 | 102 | 4.08 | 0.97 |
| **PE/PP** | 3095 | 306 | 4.02 | 0.97 |

A visual representation of the model performance within this first evaluation exercise is part of figs. 8a and 8b.

**Claims**

1. A computer implemented method for predicting the ductile-to-brittle transition temperature (DBTT) of a test polymer composition, the method comprising the following steps:

   a) training a machine learning algorithm with training polymer compositions by mapping data sets extracted from impact curves of the training polymer compositions to values indicative of ductile-to-brittle transition temperatures of the training polymer compositions;
   b) feeding the trained algorithm, as an input, with corresponding data set(s) extracted from at least one impact curve of the test polymer composition; and
   c) receiving, as an output, a value indicative of the ductile-to-brittle transition temperature of the test polymer composition.

2. The method of claim 1, wherein each of the training polymer compositions and the test polymer composition is a polyolefin composition, the polyolefin composition preferably comprising from 50 to 100 wt.-%, more preferably from 60.0 to 99.8 wt.-%, most preferably from 70 to 95 wt.-% of a polyolefin, wherein the polyolefin is most preferably selected from the group consisting of recycled or virgin polypropylene, recycled or virgin polyethylene, or a blend thereof.

3. The method of claim 1 or 2, wherein the test polymer composition and/or the training polymer compositions is/are polyolefin compositions comprising at least one or more impact modifier, wherein the total content of the impact modifier is preferably from 0.1 to 50 wt.-%, more preferably from 2.0 to 45 wt.-%, based on the total weight of the test polymer composition and/or the training polymer compositions.

4. The method of one of the preceding claims, wherein the training step a) comprises a first training step and a second training step and wherein the method comprises at least one additional step selected from cross validation and hyperparameter optimization, the at least one additional step being preferably performed after the first training step and before the second training step.

5. The method of one of the preceding claims, wherein the machine learning algorithm is based on random forest regression or a convolutional neural network.

6. The method of one of the preceding claims, wherein the training step a) is carried out with data sets extracted from at least 50, preferably at least 100, in particular at least 1000 impact curves of training polymer compositions, and/or wherein the training step a) is carried out with data sets extracted from impact curves of at least 15, preferably at least 30, in particular at least 40 different training polymer compositions.

7. The method of one of the preceding claims, wherein each of the impact curves of the training polymer compositions and the at least one impact curve of the test polymer composition have been obtained by instrumented experimentation, preferably in a temperature range from -70 °C to +60 °C.

8. The method of one of the preceding claims, wherein each of the impact curves of the training polymer compositions and/or the at least one impact curve of the test polymer composition is/are force-displacement curve(s) obtained according to an impact test or a curve derived thereof, wherein the impact test is preferably selected from the following tests:

   • Charpy impact test according to ISO 179-2:2020;
   • Izod impact test according to ASTM D256 or ISO180; and
   • puncture impact test according to ISO 6603-2:2002-04.

9. The method of one of the preceding claims, wherein the data sets extracted from impact curves of training polymer compositions and the data set(s) extracted from the at least one impact curve of the test polymer composition comprise or consist of:

   i) force-displacement data pairs as read from the respective impact curve; or
   ii) values of curve characteristics derived from the impact curve, wherein the curve characteristics are preferably selected from the group consisting of:

      • the maximum force;
      • displacement at maximum force;
      • area under curve representative of energy;
      • highest deflection;
      • number of local force maxima;
      • average force;
      • average deflection;
      • median force;
      • median deflection;
      • skew of the distribution of force values;
      • kurtosis of the distribution of force values;
      • skew of the distribution of energy values;
      • kurtosis of the distribution of energy values;
      • standard deviation of force values;
      • variance of force values; and combinations thereof.

10. The method of one of the preceding claims, wherein each of the data sets extracted from impact curves of training polymer compositions and/or the data set(s) extracted from the at least one impact curve of the test polymer composition comprise(s) or consist(s) of curve characteristics derived from the impact curve and comprises more than one value per curve characteristic, each value being derived from a different segment of the impact curve.

11. The method of one of the preceding claims, wherein each of the data sets extracted from impact curves of training polymer compositions has been reduced by a pattern recognition technique, preferably by hierarchical cluster analysis or by principal component analysis before the training step a).

12. The method of one of the preceding claims, wherein the values indicative of ductile-to-brittle transition temperatures of the training polymer compositions and the value indicative of the ductile-to-brittle transition temperature of the test polymer composition are an absolute temperature or a temperature difference.

13. The method of one of the preceding claims, wherein the value indicative of the ductile-to-brittle transition temperature is used for controlling a process in a development of application-tailored polymer composition.

14. A non-transitory computer readable storage medium for tangibly storing computer program instructions capable of being executed by a processor, the computer program instructions defining the steps of one of the preceding method claims.

15. Use of impact curves and values indicative of ductile-to-brittle transition temperatures of polymer compositions for training a machine learning algorithm.

100

```
                    ┌─────────────┐
                    │    Start    │
                    └──────┬──────┘
                           │
       ┌ ─ ─ ─ ─ ─ ─ ─ ─ ─ ▼ ─ ─ ─ ─ ─ ─ ─ ─ ┐      101
         Feature Engineering
       └ ─ ─ ─ ─ ─ ─ ─ ─ ─ ┬ ─ ─ ─ ─ ─ ─ ─ ─ ┘
                           │                         111
                    ┌──────▼──────────────┐
                    │ Mapping of input data│
                    └──────┬──────────────┘
                           │                         112
       ┌ ─ ─ ─ ─ ─ ─ ─ ─ ─ ▼ ─ ─ ─ ─ ─ ─ ─ ─ ┐
         Cross-Validation
       └ ─ ─ ─ ─ ─ ─ ─ ─ ─ ┬ ─ ─ ─ ─ ─ ─ ─ ─ ┘
                           │                         121
                    ┌──────▼──────────────┐
                    │ Feeding with data related to │
                    │  test polymer composition    │
                    └──────┬──────────────┘
                           │                         122
                    ┌──────▼──────────────┐
                    │  Receiving output    │
                    └──────┬──────────────┘
                           │
                    ┌──────▼──────┐
                    │     End     │
                    └─────────────┘
```

110

120

## Fig. 1

Fig. 2

Fig. 3a

Fig. 3b

Fig. 4

**Fig. 5**

AlexNet
Multivariat
Time-Series
1D

768x1

Dropout(0.5)
Linear(5376, 1024)
BatchNorm1d(1024, momentum=0.1)
ReLU
Dropout(0.25)
Linear(1024, 1024)
BatchNorm1d(1024, momentum=0.1)
ReLU
Linear(1024, 1)

ΔT

Fig. 6

| Dataset $n^{train}$ $n^{Test}$ | | | | | |
|---|---|---|---|---|---|
| $n^{train}/5$ | $n^{train}/5$ | $n^{train}/5$ | $n^{train}/5$ | $n^{train}/5$ | $n^{Test}$ |

combined predictions
on unseen data (median)

| | | | | | |
|---|---|---|---|---|---|
| Train | Train | Train | Train | Valid. | → Model1 |
| Train | Train | Train | Valid. | Train | → Model2 |
| Train | Train | Valid. | Train | Train | → Model3 |
| Train | Valid. | Train | Train | Train | → Model4 |
| Valid. | Train | Train | Train | Train | → Model5 |

prediction standard deviation
as uncertainty

**Fig. 7**

**Fig. 8a**

**Fig. 8b**

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | NAZARI A. ET AL: "Modeling ductile to brittle transition temperature of functionally graded steels by ANFIS", APPLIED MATHEMATICAL MODELLING, vol. 36, no. 8, 20 November 2011 (2011-11-20), pages 3903-3915, XP093050243, GB ISSN: 0307-904X, DOI: 10.1016/j.apm.2011.11.032 * the whole document * ----- | 1-15 | INV. G16C60/00 G06N3/00 |
| A | SELEZNEVA M. ET AL: "The brittle-to-ductile transition in tensile and impact behavior of hybrid carbon fibre/self-reinforced polypropylene composites", COMPOSITES PART A, ELSEVIER, AMSTERDAM, NL, vol. 109, 23 February 2018 (2018-02-23), pages 20-30, XP085389088, ISSN: 1359-835X, DOI: 10.1016/J.COMPOSITESA.2018.02.034 * the whole document * ----- | 1-15 | TECHNICAL FIELDS SEARCHED (IPC) G16C G06N |
| A | MICCIO L. A. ET AL: "From chemical structure to quantitative polymer properties prediction through convolutional neural networks", POLYMER, ELSEVIER, AMSTERDAM, NL, vol. 193, 122341, 2 March 2020 (2020-03-02), pages 1-7, XP086113974, ISSN: 0032-3861, DOI: 10.1016/J.POLYMER.2020.122341 [retrieved on 2020-03-02] * the whole document * ----- -/-- | 1-15 | |

The present search report has been drawn up for all claims

| Place of search | Date of completion of the search | Examiner |
|---|---|---|
| The Hague | 2 June 2023 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 22 21 5223

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BHOWMIK R. ET AL: "Prediction of the specific heat of polymers from experimental data and machine learning methods", POLYMER, vol. 220, 123558, 22 February 2021 (2021-02-22), pages 1-9, XP093050934, AMSTERDAM, NL ISSN: 0032-3861, DOI: 10.1016/j.polymer.2021.123558 * the whole document *  ----- | 1-15 | |
| A | CRAVERO F. ET AL: "Computer-aided design of polymeric materials: Computational study for characterization of databases for prediction of mechanical properties under polydispersity", CHEMOMETRICS AND INTELLIGENT LABORATORY SYSTEMS, ELSEVIER SCIENCE PUBLISHERS B.V. AMSTERDAM, NL, vol. 191, 19 June 2019 (2019-06-19), pages 65-72, XP085754432, ISSN: 0169-7439, DOI: 10.1016/J.CHEMOLAB.2019.06.006 [retrieved on 2019-06-19]  ----- | 1-15 | TECHNICAL FIELDS SEARCHED (IPC) |

-/--

The present search report has been drawn up for all claims

| Place of search | Date of completion of the search | Examiner |
|---|---|---|
| The Hague | 2 June 2023 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 4

Europäisches
Patentamt

European
Patent Office

Office européen
des brevets

## EUROPEAN SEARCH REPORT

Application Number

EP 22 21 5223

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | STOLL A. ET AL: "Machine learning for material characterization with an application for predicting mechanical properties", GAMM-MITTEILUNGEN, vol. 44, no. 1, 1 March 2021 (2021-03-01), XP055942649, Hoboken, USA ISSN: 0936-7195, DOI: 10.1002/gamm.202100003 Retrieved from the Internet: URL:https://onlinelibrary.wiley.com/doi/full-xml/10.1002/gamm.202100003> * the whole document * | 1-15 | |
| A | DING X. ET AL: "Predictions of macroscopic mechanical properties and microscopic cracks of unidirectional fibre-reinforced polymer composites using deep neural network (DNN)", COMPOSITE STRUCTURES, ELSEVIER SCIENCE LTD, GB, vol. 302, 116248, 23 September 2022 (2022-09-23), pages 1-13, XP087196321, ISSN: 0263-8223, DOI: 10.1016/J.COMPSTRUCT.2022.116248 [retrieved on 2022-09-23] * the whole document * | 1-15 | |

TECHNICAL FIELDS SEARCHED (IPC)

-/--

The present search report has been drawn up for all claims

| Place of search | Date of completion of the search | Examiner |
|---|---|---|
| The Hague | 2 June 2023 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

**EUROPEAN SEARCH REPORT**

Application Number

EP 22 21 5223

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TORRES J. ET AL: "Mechanics of the small punch test: a review and qualification of additive manufacturing materials", JOURNAL OF MATERIAL SCIENCE, KLUWER ACADEMIC PUBLISHERS, DORDRECHT, vol. 56, no. 18, 17 March 2021 (2021-03-17), pages 10707-10744, XP037523888, ISSN: 0022-2461, DOI: 10.1007/S10853-021-05929-8 [retrieved on 2021-03-17] * the whole document * ----- | 1-15 | |

TECHNICAL FIELDS SEARCHED (IPC)

The present search report has been drawn up for all claims

| Place of search | Date of completion of the search | Examiner |
|---|---|---|
| The Hague | 2 June 2023 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 390 943 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021110690 A1

- US 10955362 B2

**Non-patent literature cited in the description**

- *CHEMICAL ABSTRACTS,* 6683-19-8
- *CHEMICAL ABSTRACTS,* 31570- 04-4
- *CHEMICAL ABSTRACTS,* 693- 36-7
- *CHEMICAL ABSTRACTS,* 11097-59-9
- *CHEMICAL ABSTRACTS,* 1592-23-0
- *CHEMICAL ABSTRACTS,* 557-05-1
- *CHEMICAL ABSTRACTS,* 60676-86-0
- *CHEMICAL ABSTRACTS,* 7631-86-9
- *CHEMICAL ABSTRACTS,* 112926-00-8
- *CHEMICAL ABSTRACTS,* 1318-74-7
- *CHEMICAL ABSTRACTS,* 1344-00-9
- *CHEMICAL ABSTRACTS,* 92704-41-1
- *CHEMICAL ABSTRACTS,* 1327-36-2
- *CHEMICAL ABSTRACTS,* 1344-95-2
- *CHEMICAL ABSTRACTS,* 1344-01-0
- *CHEMICAL ABSTRACTS,* 52829-07-9
- *CHEMICAL ABSTRACTS,* 1843-05-6
- *CHEMICAL ABSTRACTS,* 532-32-1
- *CHEMICAL ABSTRACTS,* 135861-56-2
- *CHEMICAL ABSTRACTS,* 97593-29-8
- *CHEMICAL ABSTRACTS,* 71786-60-2
- *CHEMICAL ABSTRACTS,* 204-393-1